# EUROPEAN PATENT SPECIFICATION

(11) **EP 0 768 091 B1**
(45) Date of publication and mention of the grant of the patent: **30.07.2003**
(21) Application number: 95116289.0
(22) Date of filing: 16.10.1995
(51) Int. Cl.: A61M 1/10

(54) **Artificial heart**
Kunstherz
Coeur artificiel

(43) Date of publication of application: 16.04.1997
(73) Proprietor: SUN MEDICAL TECHNOLOGY RESEARCH CORPORATION, Suwa-shi, Nagano-ken 392-0004 (JP)
(72) Inventor: Yamazaki, Kenji, Koganei-shi, Tokyo (JP); Mori, Toshio, Chino-shi, Nagano-ken (JP); Higuchi, Kouji, Hirookayoshida, Shiojiri-shi, Nagano-ken (JP)
(74) Representative: Weber, Joachim, Dr.

(56) References cited:
- EP-A- 0 659 443
- WO-A-85/01436
- WO-A-89/04644
- US-A- 4 817 586
- US-A- 4 898 518

## Description

This invention relates to an artificial organ, for example an auxiliary artificial heart of an embedded type, embedded in the left or right ventricle of the heart in a human body, and operated at high reliability by preventing body fluids such as blood from adversely entering the artificial heart.

Conventional artificial hearts are of a diaphragm type, of a sack type, of an axially symmetric type, of a centrifugal type, of a pusher plate type or the like. Each of these conventional artificial hearts delivers blood in place of a human heart or by bypassing it.

Recently, there has been developed an auxiliary artificial heart which is embedded in a ventricle of a human heart and has the tip end of a nozzle passing through an aorta valve or the like such that blood is delivered from the ventricle into an aorta through the nozzle. The artificial heart has such a structure that it does not suppress any function of the human heart when it is provided in the human heart and it delivers additional blood into the aorta when blood pumped out from the human heart due to its beats is insufficient. The blood is delivered not only by the artificial heart but also by the pulsing human heart due to its beats. Even if the operation of the artificial heart happens to stop, blood is delivered by the human heart due to its beats.

Naturally, the volume of the part of the artificial heart which is inserted in a ventricle of the human heart must be smaller than the volume of the human heart when it is contracted most. Such an artificial heart has a pump body comprising a cylindrical axial-flow pump section, a nozzle section provided on its distal end and a driving section provided on the proximal end of the axial-flow pump section. The cardiac apex of a ventricle of a human heart is cut and a short cylindrical cardiac apex ring is embedded therein. The pump section and the nozzle section are inserted in the ventricle through the cardiac apex ring, and the distal end of the nozzle section is inserted in an aorta through its aorta valve or the like. The driving section which has a large volume is embedded in a portion of the thorax which is outside of the human heart.

The artificial heart has the following problem in connection with the function of a shaft-sealing mechanism provided between the pump section and the driving section. With artificial heart, a motor and other elements are housed in the driving section, and the rotor of the pump section is driven via driving shaft extending from the driving section to the pump section. Blood applied with a blood pressure flows through the pump section. In this state, blood is not allowed to enter the space in the driving section. If blood enters the space defined in the driving section, coagulation of blood occurs and the operation of the motor stops.

It is necessary to provide, between the driving section and the pump section, a sealing mechanism for sealing the driving shaft in liquid tight state in order to prevent blood from entering the interior of the driving section. Since, however, the artificial heart is embedded in a human body, the artificial heart must be operated for a long time without maintenance. It is not easy with the present technology to provide a shaft-sealing mechanism with which perfect sealing is maintained for a long time.

In order to dissolve the above identified problems, the inventors of the present invention have developed an artificial heart provided with an improved sealing mechanism for the driving shaft thereof. Patent Application No. 94105896.8 (EP-A-0 629 412) filed on April 15, 1994 by the applicant of the present patent application discloses such an artificial heart. An artificial heart disclosed in the above patent application is designed to be inserted in a ventricle of a human heart, including a cylindrical cardiac apex ring embedded in the cardiac apex of the human heart by cutting the cardiac apex, and a main body of the artificial heart comprising a cylindrical axial-flow pump section inserted in the ventricle of the human heart through the cardiac apex ring, a nozzle section extending outward from the distal end of the pump section through the aorta valve of the human heart and a driving section provided on the proximal end of the pump section and disposed outside (or externally) of the human heart, for driving the pump section through a driving shaft.

Between the driving section and the pump section is provided a sealing mechanism for maintaining the driving shaft in a liquid tight state so as to prevent blood from entering the interior of the driving section from the pump section. The sealing mechanism defines a sealing liquid chamber surrounding the driving shaft at the driving section and a sealing liquid is filled in the sealing liquid chamber.

The sealing liquid includes a physiological sodium chloride solution or an anticoagulant such as heparin, and the sealing liquid chamber communicates with a sealing liquid bag made of flexible material, filled with the sealing liquid and embedded in the human body.

The sealing mechanism is provided with an oil seal made of elastic material, closely fitted on the outer peripheral surface of the driving shaft due to its elastic deformation and forming a lubricating film of the sealing liquid between the peripheral surface of the driving shaft and the oil seal.

The pump section is driven by a motor or the like driving unit housed in the driving section. The pump section sucks blood from a ventricle of a human heart and discharges it into an aorta from the nozzle section of the distal end of the pump section by bypassing the aorta valve or the like. Thus, blood is delivered to the aorta not only by the beats of the human heart but also by means of the artificial heart. The artificial heart supplements insufficient amount of blood which is not provided by the human heart, whereby it is ensured that the necessary amount of blood can be delivered. The volume of the pump section is smaller than the volume of the ventricle of the human heart when it contract most so as not to prevent natural beats of the human heart.

The sealing mechanism prevents blood from entering the driving section from the pump section. In this case, the sealing mechanism defines a sealing liquid chamber at the driving section, and a sealing liquid such as a physiological sodium chloride solution is filled in the sealing liquid chamber. Sealing and lubrication of the sealing mechanism are ensured by the sealing liquid, and blood is securely prevented from entering the interior of the driving section from the sealing mechanism.

Even if the sealing mechanism is deteriorated and blood enters the mechanism, the blood which has entered the mechanism is mixed with the sealing liquid. Thus, blood is not coagulated and does not prevent the smooth operation of the artificial heart.

The sealing mechanism is provided with an oil seal which forms a lubricant film of the dealing liquid between the oil seal and the outer peripheral surface of the driving shaft and is elastically closely fitted on the outer peripheral surface of the driving shaft for securely preventing the entrance of blood such that the oil seal is not worn and has high durability. The oil seal can be designed such that the lubricant film formed between the oil seal and the outer peripheral surface of the driving shaft delivers blood in only one direction toward the pump section. This structure prevents blood from entering the interior of the driving section.

The sealing liquid chamber communicates with the sealing liquid bag embedded in the human thorax or the like. A sealing liquid is supplemented from the sealing liquid bag and thus can be supplied to the sealing liquid chamber for a long time.

The driving shaft uses a dynamic pressure bearing made of ceramic material operated in the sealing liquid. A coating film is formed between the sliding surfaces due to the dynamic pressure of the sealing liquid, thereby reducing rotational resistance of the bearing and preventing its wear, leading to high reliability.

When the driving shaft is rotated, the dynamic pressure bearing generates dynamic pressure. The dynamic pressure provided a liquid seal between the sealing liquid chamber and the driving section. The sealing liquid is thereby prevented from flowing from the sealing liquid chamber into the driving section.

The dynamic pressure bearing, which is mounted on the distal end portion of the driving shaft, supplied the sealing liquid to the oil seal. Hence, the sealing liquid circulates in the artificial heart, preventing foreign body from depositing.

On the other hand, the above described sealing mechanism of artificial heart has room for improvement in order to relieve the burden on the part of the patient who carries such an artificial heart. More specifically, since the artificial heart is embedded in the patient's proper heart and remains there for use, the patient's chest has to be opened and the artificial heart has to be taken out for servicing or replacement, imposing a large burden to the patient. Thus, an artificial heart is required to operate reliably for a very long period of time without servicing.

While the above described sealing mechanism comprising an oil seal operates satisfactorily in terms of the sealing effect, it remains to be improved particularly in the following aspects.

Firstly, the durability of the material used in the sealing mechanism. The oil seal is typically made of an elastic material such as synthetic rubber. Of course, there are a number of elastic materials having a sufficient durability. However, as for synthetic rubber, the period guaranteed for the initial physical properties of synthetic rubber is normally specified by the supplier for each product, starting form the cure date of the product.

This means that the artificial heart comprising an oil seal of the above described type for its sealing mechanism and embedded in a patient has to be taken out of the patient by cutting his or her chest before the guaranteed period expires. Thus, a material having such a limited guaranteed period may preferably not be used for the sealing mechanism of an artificial heart.

Secondly, the period until the next supply of sealing liquid to an embedded artificial heart should be made as long as possible. The patient has to visit the hospital and see the doctor who carries out the operation of supplying sealing liquid in order to prevent any possible contamination by microbes of the sealing liquid contained in the bag of the patient. Such a visit on the part of the patient would be particularly burdensome when the sealing liquid bag is embedded in the patient. Thus, the period until the next supply of sealing liquid should be made as long as possible, although this period can be prolonged by reducing the consumption rate of sealing liquid by the patient.

If an oil seal is used as described above, the consumption rate of sealing liquid can rise after a long use. A thin film of sealing liquid is normally formed between the oil seal and the sealing surface of the driving shaft to maintain its lubricating and sealing effect. However, some protein in the blood can permeate into the thin film by diffusion and become coagulated by the heat generated by the friction between the oil seal and the sealing surface. While the coagulated protein takes the form of granules and mostly driven out of the sealing mechanism with sealing liquid being supplied and eventually dispersed into the blood, part of the granules of coagulated protein can adhere to the surface of the oil seal. Now, since the lip section of the oil seal is apt to be enlarged by small force, granules of coagulated protein can easily be deposited there to broaden the gap between the oil seal and the sealing surface of the driving shaft and consequently raise the flow rate of sealing liquid running through the gap. The net result will be that the time up to the next supply of sealing liquid is curtailed to put more burden on the patient.

Thirdly, the reliability of the sealing mechanism has to be improved. An artificial heart of the type under consideration and particularly the operation of the sealing mechanism can be examined only indirectly by checking the consumed volume and the level of contamination of the sealing liquid. If it is found that too much sealing liquid has been consumed unexpectedly and/or the sealing liquid has been contaminated, if to a small extent, replacement of the artificial heart may have to be considered for the sake of safety. This by turn will increase the burden on the part of the patient.

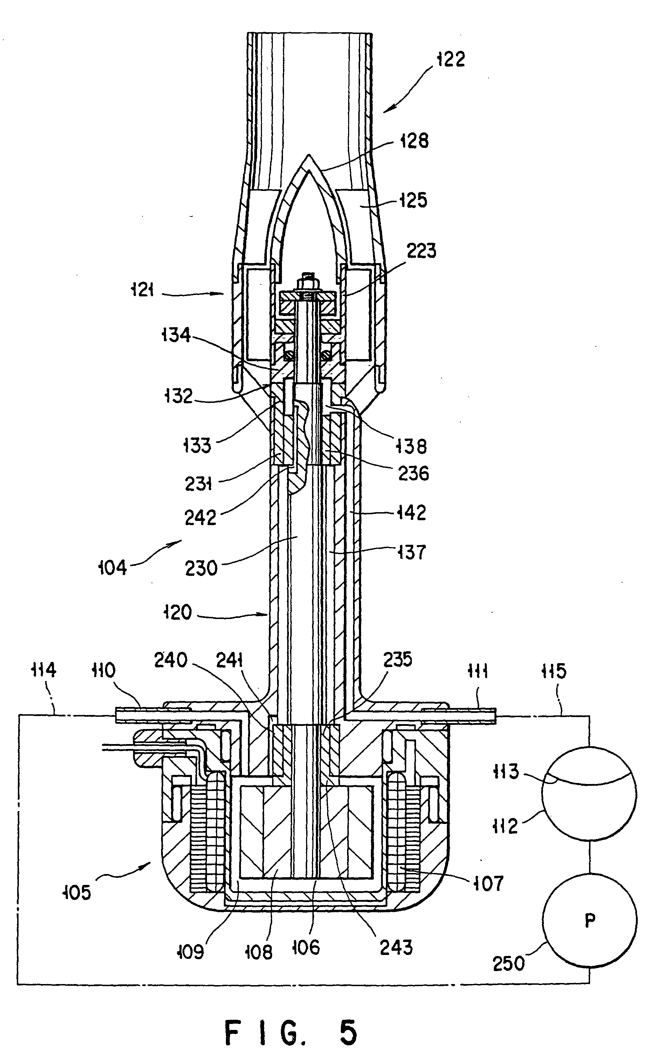

WO 85/01436 discloses a blood pump comprising a rotor and a stator which co-operate to convey a blood stream to the pump. As shown in Fig. 5, there is provided a drive shaft. The rotor is positioned with respect to the stator by a pair of spherical bearings.

WO 89/04644 discloses a miniature high-speed intravascular blood pump according to the preamble portion of claim 1.

In view of the above circumstances, it is therefore a first object of the present invention to provide an artificial heart comprising a sealing mechanism made of a material having a practically endless service life to make the artificial heart free from maintenance for a prolonged period of time. A second object of the invention is to provide an artificial heart comprising a sealing mechanism that is protected against degradation of its sealing performance due to a deposit of coagulated protein. A third object of the invention is to provide an artificial heart having an improved reliability.

These objects are solved by an artificial organ according to claim 1. The sub-claims contain preferred embodiments of the invention.

According to the present invention, the above objects and other object of the invention are achieved by providing an artificial heart comprising a sealing mechanism for the driving shaft of the artificial heart that includes a mechanical seal. The mechanical seal includes a fixed side seal ring and a rotary side follow ring with the ends of the seat ring and follow ring in close contact with each other so as to rotate freely and also contains a pressing mechanism for pressing the follow ring against a seat ring at a specific pressure.

An artificial heart according to the invention also comprises a sealing liquid chamber surrounding the driving shaft and arranged between the mechanical sealing and the driving section, the sealing liquid chamber being filled with a sealing liquid. An artificial heart according to the invention additionally comprises pump means for feeding the sealing liquid from the sealing liquid chamber to the mechanical seal.

An artificial heart according to the invention and having the above described configuration does not comprise an oil seal and other components made of synthetic rubber in the sealing mechanism that limits the service life of the artificial heart so that the artificial heart is practically free from maintenance for a prolonged period of time.

In the mechanical seal, the ends of the seat ring and follow ring in close contact with each other so as to rotate freely and provide sealing surfaces. Thus, a thin film of sealing liquid is formed between the sealing surfaces to produce a lubricating and sealing effect. With this arrangement, any foreign objects that may exist between the sealing surfaces are subjected to centrifugal force as the follow ring rotates. In other words, if protein and other substances permeate into the gap between the sealing surfaces and become coagulated to give rise to granules thereof, they are driven out from the mechanical seal and dispersed into the blood running through the heart by the generated centrifugal force. Since the granules of coagulated protein has a very small diameter and at the same time are very small in quantity, they would not adversely affect the host if they are dispersed into the blood.

Of the above mechanical seal, the sliding surfaces of the seat ring and the follow ring are polished and held in close contact with each other and the follow ring is pressed against the seat ring under high pressure to preclude any possibility of adhesion of coagulated protein granules to the sliding surfaces and, therefore, the gap, if any, between the sliding surfaces would not be broadened by adhering coagulated protein granules. Thus, the consumption rate of sealing liquid would not rise with time and the period between any two successive supplies of sealing liquid would not be shortened to increase the burden on the part of the patient.

Additionally, since the sealing mechanism of an artificial heart according to the invention essentially does not contain any synthetic rubber to improve its reliability in terms of abrasion and wear so that the artificial heart can enjoy a practically endless service life to the patient's benefit.

In a preferred embodiment of the invention, the driving shaft, the bearing tube of the driving shaft, the seat ring and other related components are made of a ceramic material, which is stable both chemically and dimensionally and highly resistant against abrasion. This makes the artificial heart even more reliable and prolongs its service life.

In another preferred embodiment of the invention, the driving shaft and the bearing section are integrally formed and the bearing section is slidably engaged with the inner peripheral surface of the bearing tube to support the driving shaft. A dynamic pressure generating groove if formed on the outer peripheral surface of the bearing section so that sealing liquid is introduced into the gap between the outer peripheral surface of the bearing section and the inner peripheral surface of the bearing tube to ensure lubrication therebetween. The dynamic pressure generating groove is asymmetrical relative to the axial direction of the driving shaft so that it operates as a pump for feeding sealing liquid axially up to the mechanical seal at a very low rate. Thus, the entire configuration of the artificial heart is very simple and hence very reliable.

This invention can be more fully understood from the following detailed description when taken in conjunction with the accompanying drawings, in which:
FIG. 1 is a cross-sectional view of an artificial heart of a first embodiment of this invention, provided in the left ventricle of a human heart;
FIG. 2 is a longitudinal cross-sectional view of an artificial heart of the embodiment of FIG. 1;
FIG. 3 is a longitudinal cross-sectional view of the bearing tube in the embodiment of FIG. 1;
FIG. 4 is a side view of the driving shaft of FIG. 1;
FIG. 5 is a longitudinal cross-sectional view of an artificial heart of a second embodiment according to the present invention;
FIG. 6 is a longitudinal cross-sectional view of the mechanical sealing mechanism section of FIG. 5; and
FIG. 7 is a longitudinal cross-sectional view of an artificial heart of a third embodiment according to the present invention.

The embodiments of this invention will be described with reference the accompanying drawings.

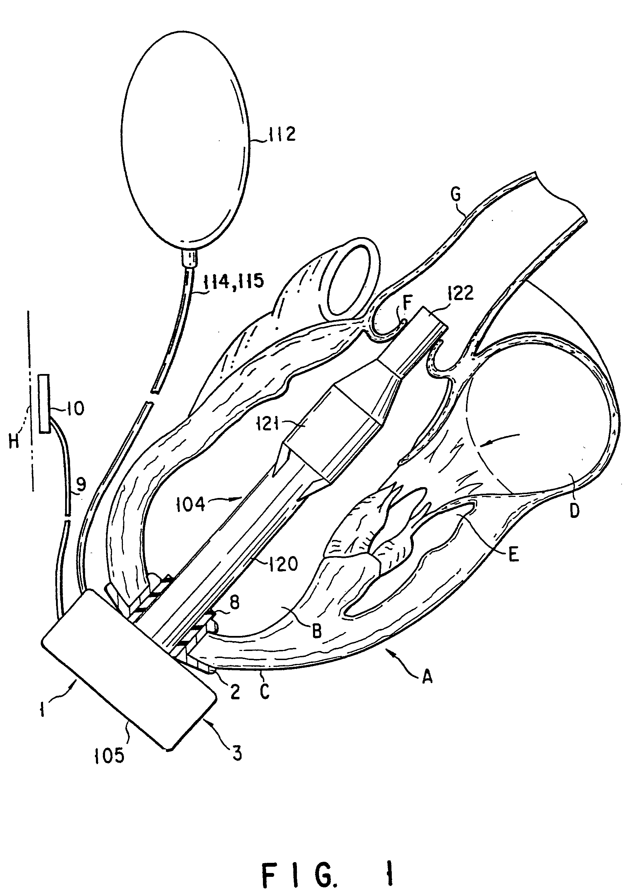

A first embodiment of this invention is shown in FIGS. 1 to 4. FIG. 1 shows an artificial heart of this invention in a state embedded in the left ventricle B of the heart A of a patient (hereinafter referred to as the "human heart"). A cardiac apex, a left atrium, a mitral valve, an aorta valve and an aorta are designated by C, D, E, F and G, respectively.

The artificial heart 1 comprises a cardiac valve ring 2 and the main body 3 of the artificial heart. The cardiac valve ring 2 is a short cylindrical member having a flange and is embedded in the human heart A through the cardiac apex C of the human heart A after the cardiac apex C has been cut. The main body 3 of the artificial heart 1 comprises a pump section 104, a nozzle section 122 provided on the distal end of the pump section 104 and a driving section 105 provided on the proximal end of the pump section 104. The pump section 104 and the nozzle section 122 are inserted in the left ventricle B through the cardiac apex ring 2, and the nozzle section 122 is further inserted in the aorta G through the central portion of the aorta valve F. Liquid tightness is ensured between the cardiac apex ring 2 and the main body 3 by means of a ordinary sealing mechanism, for example, a sealing member 8.

The pump section 104 is a relatively small cylindrical member and it has a smaller volume than the volume of the left ventricle B when it contracts most so as not to prevent natural beats of the human heart A. In the pump section 104 is housed a small axial-flow pump which is driven by a motor provided in the driving section 105. The pump section 104 sucks blood from the left ventricle B at a suction port formed in the outer peripheral surface of the section 104 and discharges the blood from the distal end of the nozzle section 122 into the aorta G with the aorta valve F bypassed.

The nozzle section 122 passing through the central portion of the aorta valve F is made of soft synthetic resin material such that it does not suppress the function of the aorta valve F and it does not injure the aorta valve F.

The driving section 105 is embedded in a portion of the thorax outside of the human heart A. In the driving section 105 are provided a motor and other elements such as electric cells and electronic control elements if they are required. The driving section 105 is connected by means of electric wires 9 to a noncontact type electrode 10 embedded in a portion of the human body close to his skin H. A necessary electric power is supplied from an external electric source (not shown) to the driving section 105 through the electrode 10.

In the driving section 105, a motor 106 is provided. The motor 106 is a canned motor where a rotor is housed in a casing filled with liquid. Numeral 107 indicate a stator coil and 108 a rotor. The spacing between the rotor 108 and the casing, or an air gap 109 is designed to allow a sealing liquid to circulate.

In the driving section 105, a sealing liquid inlet 110 and a sealing liquid outlet 111 are made. They are connected to a seal liquid bag 112 via flexible circulation tubes 114, 115, respectively. Then, the sealing liquid circulates between the sealing liquid bag 112 and the inside of the artificial heart. In the sealing liquid bag 112, there is provided a filter, which removes foreign matter mixed in the circulating sealing liquid.

The pump section 104 contains a thin-wall, cylindrical tube section 120 and a casing section 121 formed at the tip portion of the tube section. In the casing section 121, a pump rotor composed of a rotor boss 123 and a plurality of rotor blades 124 provided on the boss so as to project from around the boss is housed. The pump rotor is rotated by the motor 106. Numeral 128 indicates a spinner that provides flow straightening. In the casing section 121, there are also provided a plurality of stator vanes 125 and a plurality of front stator vanes also serving as a stay for the casing section 121.

On the base end side of the casing section 121, a fluid inlet 127 is made, through which the blood in the left ventricle of the heart is sucked. The blood is discharged from the distal end of a nozzle section 122. The nozzle section 122 is inserted in the aorta through the aorta valve of the heart.

The number of the stator vanes 125, 126 and that of rotor blades 124 are set at values that do not contain the common factors or integral multiples of the common factors, that is, at prime factors with respect to each other, which thereby prevents the resonance of pulsation of blood sent by rotation of the rotor blades 124.

Explained next will be the driving shaft of the artificial heart and its sealing mechanism in the present embodiment. In the figure, numeral 130 indicates a driving shaft. The base end portion of the driving shaft 130 is connected to the rotor 108 of the motor 106. The tip end portion of the shaft is connected integrally to the rotor boss 123 of the pump rotor, which is driven via the driving shaft 130. In the tube section 120 of the pump section 104, a cylindrical bearing tube 131 is housed. The driving shaft 130 is inserted in the bearing tube 131.

At the tip portion of the bearing tube 131, a mechanical sealing mechanism 132 acting as a sealing mechanism is provided. The mechanical sealing mechanism 132 presents the blood in the left ventricle of the heart from entering the inside the artificial heart.

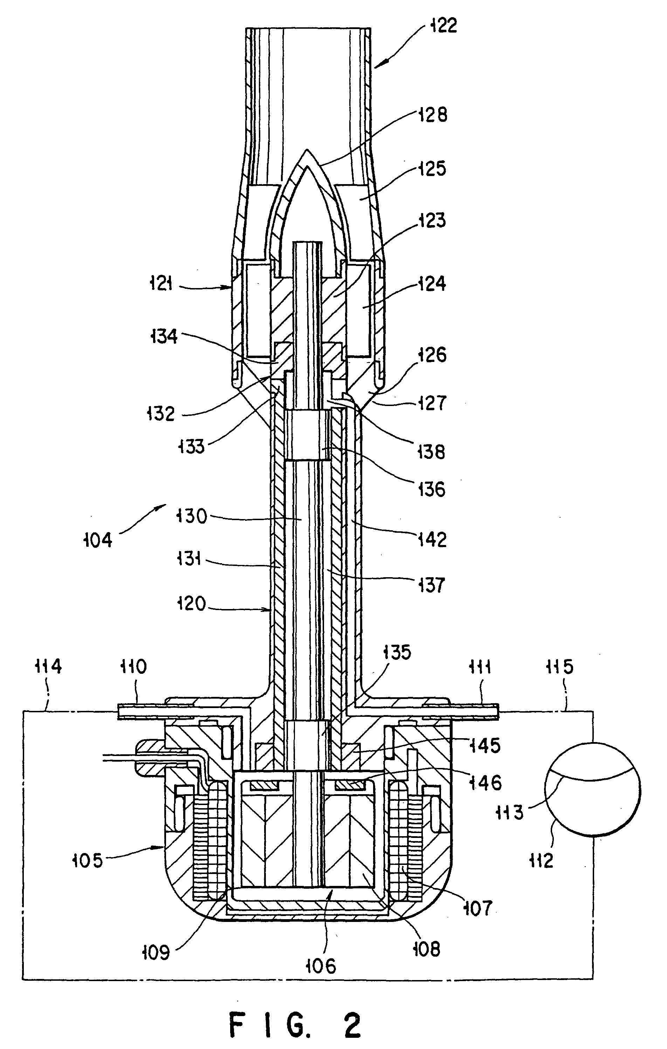

The construction of the mechanical sealing mechanism 132 will be described with reference to FIGS. 2 to 4. At the tip portion of the bearing tube 131, a flange-like fixed-side seat ring 133 is formed. The end of the seat ring 133 is worked precisely so as to have a smooth surface perpendicular to the rotation axis of the driving shaft 130 and provides a sealing surface. A rotary-side follow ring 134 is in close contact with the sealing surface of the seat ring 133 so as to rotate freely, thereby maintaining the effect of sealing. The follow ring 134 has a disk shape and is provided integrally at the tip portion of the driving shaft 130 so as to be precisely perpendicular to the rotation axis of the driving shaft.

Furthermore, at the base end portion and tip portion of the driving shaft 130, bearing sections 135 and 136 are integrally formed, respectively. These bearing sections 135, 136 are cylindrical and supported by the inner surface of the bearing tube 131 so as to rotate freely. The outer surface of these bearing sections 135, 136 and the inner surface of the bearing tube 131 are worked precisely. These bearing sections 135, 136 and bearing tube 131 support the driving shaft 130 precisely so as to rotate freely.

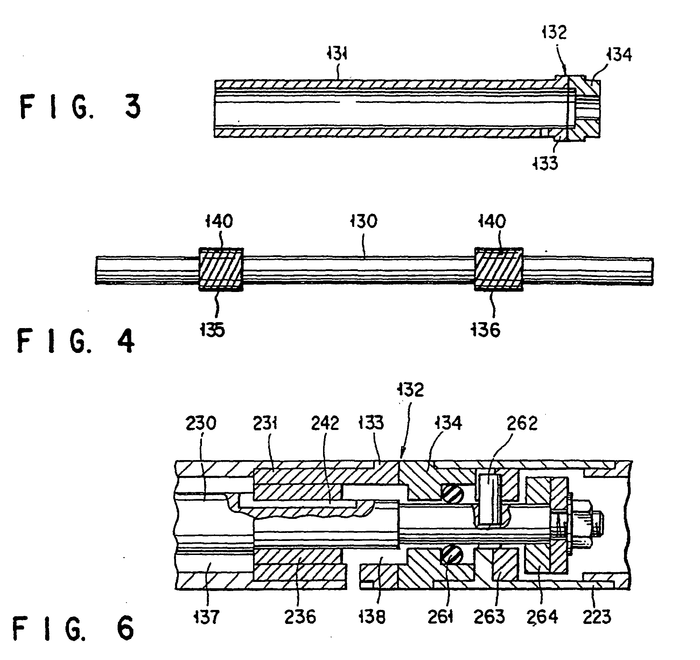

As shown in FIG. 4, spiral shallow dynamic-pressure grooves 140 are made in the outer surface of these bearing sections 135, 136. These dynamic-pressure grooves 140, when the driving shaft 130 rotates, introduces the sealing liquid into the spacing between the outer surface of these bearing sections 135, 136 and the inner surface of the bearing tube 131, thereby assuring the lubrication between them, and allows the sealing liquid to flow toward the tip portion at a specific rate. Therefore, these bearing sections 135, 136 and bearing tube 131 constitute a one-way dynamic-pressure bearing that functions as both of a bearing for supporting the driving shaft 130 and a pump for feeding the sealing liquid.

Furthermore, the driving shaft 130 is not restricted in the direction of the axis with respect to the bearing tube 131, but can move freely in the axis direction. In the driving section 105, a ring-shaped permanent magnet 145 is provided on the fixed-side of the housing of the driving section. A ring-shaped permanent magnet 146 is also provided on the rotor 108 of the motor 106, that is, on the driving shaft 130 side. These permanent magnets 145, 146 face each other with a specific distance between them. The polarity of these permanent magnets 145, 146 is set so that they may repel one another in the axis direction. The repulsion of these permanent magnets 145, 146 actuates the driving shaft 130 so that the shaft may move toward the base end, thus pressing the follow ring 134 on the driving shaft 130 against the seat ring 133, thereby maintaining the sealing effect of the mechanical sealing mechanism 132.

The spacing between the bearing tube 131 and the driving shaft 130 is designed to be sealing liquid chambers 137, 138 through which the sealing liquid circulates. The sealing liquid chamber 138 is isolated from the outside, that is, the blood passageway in left ventricle of the heart by the mechanical sealing mechanism 132, thereby maintaining the effect of sealing.

In the outer portion of the tube section 120 of the pump section 104, a circulation passage 142 is made. The tip portion of the circulation passage 142 is connected to the sealing liquid chamber 138 and its base end portion is connected to the sealing liquid outlet 111. Therefore, the sealing liquid flows from the sealing liquid bag 112 through the circulation tube 114 and sealing liquid inlet 110 into the air gap 109 of the motor 106. Then, by the pumping action of the one-way dynamic-pressure bearing composed of the bearing sections 135, 136 and bearing tube 131, the sealing liquid is sent through the sealing liquid chambers 137, 138 toward the back of the mechanical sealing mechanism 132 at a specific pressure. The sealing liquid fed to the sealing liquid chamber 138 is returned to the sealing liquid bag 112 via the circulation passage 142, sealing liquid outlet 111, circulation tube 115, and filter 113, and circulates in this route.

The function and advantage of the first embodiment described above are as follows. First, with the one-way dynamic-pressure bearings 135, 136 feed the sealing liquid to the sealing liquid chamber 138 at a specific pressure. The sealing liquid forms a thin film of 0.5 to 1.0 µm in thickness between the seat ring 133 of the mechanical sealing mechanism 132 and the sealing surface of the follow ring 134. The thin film effects lubrication and sealing between the seat ring and the follow ring. This presents blood from entering the sealing liquid chamber 138, or the inside of the artificial heart.

When the artificial heart is actually used, protein in the blood, a little though, may enter due to diffusion. The entered protein is coagulated by hearing due to the sliding friction between the seat ring and follow ring that rotate relatively with respect to each other. Particles of the coagulated protein are discharged outward under the influence of the centrifugal force of the follow ring 134 that is rotating, and are washed away by the blood flowing outside the mechanical sealing mechanism. The particles of the coagulated protein are so small that they have no adverse effect on the human body even if diffusing in the blood.

Although the coagulated protein may cohere at the inner peripheral edge portions of the seat ring 133 and follow ring 134, such protein particles will be washed away by the sealing liquid circulating inside the mechanical sealing mechanism 134, or inside the sealing liquid chamber 138. Those protein particles are captured by the filter 113 in the sealing liquid bag 112, so that the sealing liquid will never be contaminated.

The first embodiment using the mechanical sealing mechanism as the sealing mechanism has the following advantages, as compared with the previous artificial heart using an oil seal as the sealing mechanism.

Although an oil seal has a simple structure and keeps excellently close contact with the driving shaft, when protein in the blood diffuses between the oil seal and the peripheral surface of the driving shaft and coagulates there, the coagulated protein will not be discharged by centrifugal force as described above because the oil seal is in contact with the peripheral surface of the driving shaft. Furthermore, since the oil seal has a high flexibility, when the coagulated protein deposits between the peripheral surface of the driving shaft and the oil seal, the internal diameter of the oil seal extends easily, increasing the spacing between the peripheral surface of the driving shaft and the oil seal. The increase in the spacing increases a flow rate of sealing liquid flowing outside through the spacing. The flow of the sealing liquid washes away the coagulated protein into the blood, resulting in an increase in the amount of sealing liquid flowing outside, or the consumption of sealing liquid. This makes it necessary to frequently supply sealing liquid to the sealing liquid bag, imposing a heavier burden on the patient.

In contrast, with the mechanical sealing mechanism, centrifugal force discharges the coagulated protein as noted earlier, so that the spacing between the seat ring and the follow ring can be kept narrow constantly. As a result, the flow of sealing liquid flowing outside is smaller and consequently the frequency of supply of the sealing liquid is lower, easing the burden on the patient.

Furthermore, the sealing liquid circulated as described above cools the mechanical sealing mechanism 132, effectively preventing part of the mechanism from being locally heated to high temperatures due to sliding friction. This prevents the blood cells from being destroyed as a result of the blood touching high-temperature portions.

In addition, such a mechanical seal has a higher durability than the oil seal, because the seat ring and follow ring are made of ceramic material or metal material.

In the above embodiment, the bearing tube 131 and driving shaft 130 constituting the mechanical sealing mechanism are formed of fine ceramic material, and the follow ring 134 is formed of carbon graphite material, they are precision ground. The above-described ceramic material is chemically stable and superior in dimensional stability. In the present embodiment, the bearing tube 131 and the seat ring 133 as well as the driving shaft 130 and the bearing sections 135, 136 are formed integrally, so that the dimensional accuracy of their assembly or the mechanical sealing mechanism is high. Consequently, the accuracy of the positional relationship, such as the perpendicularity or concentricity of the seat ring 133 and follow ring 134 with respect to the rotation axis, is high, assuring a high reliability. Since the follow ring 134 is formed of carbon graphite, it matches the seat ring 133 well. Additionally, the carbon graphite has self-lubricating properties, the coefficient of friction is low.

The materials for these members are not restricted to what have been described above, but may be suitable combinations of various types of ceramic, carbon graphite, composite material, metal material, and others.

In the first embodiment, the follow ring 134 is pressed against the seat ring 133 in the mechanical sealing mechanism 132 by magnetic repulsion of the permanent magnets 145, 146, resulting in a simpler configuration and a higher reliability. The urging pressure may come from the attraction between the permanent magnets.

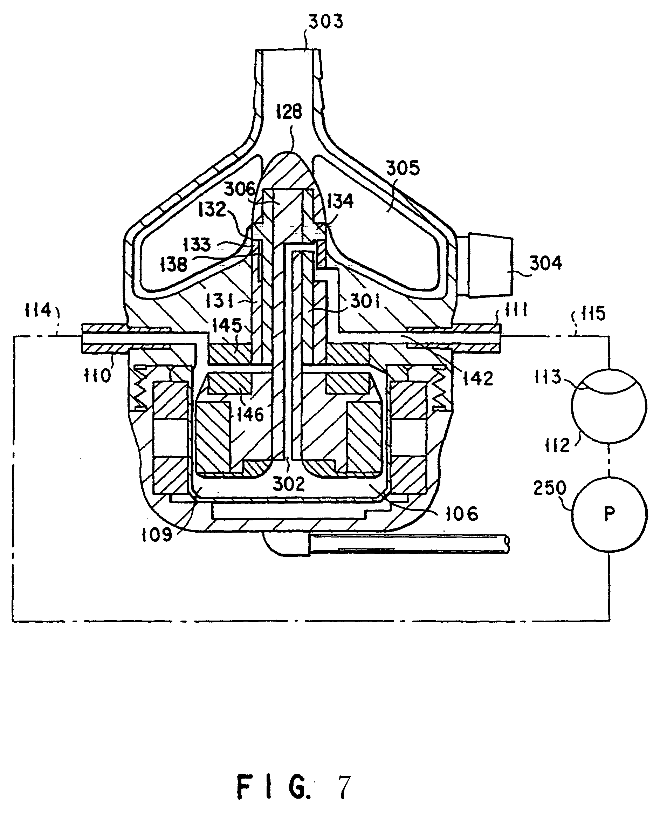

FIGS. 5 and 6 show a second embodiment of the present invention. The second embodiment is the same as the first embodiment except for part of the mechanical sealing mechanism and part of the circulation route of the sealing liquid.

Specifically, in the present embodiment, a driving shaft 230 and bearing members 235, 236 are formed into separate members. The bearing member 235 on the base end side is supported by a bearing sleeve 240, and the bearing member 236 on the tip end side is supported by a short bearing tube 231. On the bearing member 235 on the base end side, a flange section 243 is formed, thereby constituting a thrust bearing.

Between the outer surface of the bearing sleeve 240 and the housing of the driving section 5, a passageway 241 is formed. A passageway 242 is also formed between the inner surface of the bearing section 236 on the tip end side and the driving shaft 230. The sealing liquid passes through these passageways and circulates through the sealing liquid chambers 137, 138. In the present embodiment, a sealing liquid circulating pump 250 is provided outside the body or inside the abdominal cavity of the patient, and the pump 250 circulates the sealing liquid.

At the tip end portion of the bearing tube 231, a flange-like seat ring 133 is formed integrally. The follow ring 134 is in close contact with the sealing surface of the end of the seat ring 133. The follow ring 134 is a member separate from the driving shaft 230. The follow ring 134 is installed on the rotor boss 223 of the pump rotor. The rotor boss 223 is designed to move freely a specific distance in the axis direction with respect to the driving shaft 230. The follow ring 134 can move to some extent in the axis and the diameter direction with respect to the driving shaft 230. An O ring provides sealing between the follow ring 134 and the driving shaft 230.

The rotor boss 223 is hollow and houses a pair of disk-shaped permanent magnets 263, 264 in it. One permanent magnet 263 is mounted on the rotor boss 223 and the other permanent magnet 264 is mounted on the driving shaft 230. The polarity of these permanent magnets 263, 264 is set so that they may repel each other. The force of repulsion presses the follow ring 134 against the end of the seat ring 133.

In the present embodiment, the driving shaft 230, rotor boss 223, and follow ring 134 can move from each other. A relative movement between them can absorb vibrations caused by their rotation. This enables the follow ring 134 to be pressed against the end of the seat ring 133 more stably, resulting in an increase in the reliability of the mechanical sealing mechanism 132.

In the embodiment, because the sealing liquid is circulated by the sealing liquid circulating pump 250, the circulating flow rate and pressure of the sealing liquid can be regulated freely. those the second embodiment are indicated by the

Additional advantages and modifications will readily occur to those skilled in the art. Therefore, the invention in its broader aspects is not limited to the specific details, and representative devices shown and described herein. Accordingly, various modifications may be made without departing from the scope of the appended claims

Moreover, FIG. 7 shows an artificial heart according to a third embodiment of the invention. This artificial heart is equipped with a centrifugal pump, and located in a cavity outside the heart of a human body or outside the cavity.

The artificial heart has an impeller 305 serving as the centrifugal pump, which is to be driven by a motor 106. Blood is introduced through an inlet nozzle 304 via a catheter, pressurized by the impeller 305, and supplied into the aorta, etc. via an output nozzle 303 and a catheter.

In the artificial heart constructed as above, a clearance between a pump portion which houses the impeller 305 and a motor portion which houses the motor 106 is sealed with a sealing mechanism 132 similar to the aforementioned one to prevent the flow of blood into the motor portion. Further, a seal liquid such as physiologic saline is circulated via a sealing liquid chamber 138 by means of a sealing liquid circulating mechanism similar to the aforementioned one.

Although the sealing mechanism 132 employed in the fourth embodiment is similar to those employed in the first and second embodiments, the former has the following structural differences since the artificial heart of the third embodiment can be made larger than the above-described embedded-type artificial heart and hence can be designed more freely in size than the same:

In the artificial heart of the third embodiment, the motor 106 and the impeller 305 are coupled to each other by means of a driving shaft 306 on which a cylindrical shaft member 301 made of ceramics is fitted. Accordingly, the cylindrical shaft member 301 rotates together with the driving shaft 306. A driven ring 134 is formed integral with an end portion of the shaft member 301 such that it tightly contacts a seat ring 133, which is formed on an end surface of a bearing tube 131, by means of the magnetic forces of permanent magnets 145 and 146. An axial sealing liquid passage 302 is formed in a center portion of the driving shaft 306, for allowing the sealing liquid to be supplied into the sealing liquid chamber 138 therethrough.

The sealing mechanism 132 employed in the artificial heart of the third embodiment has the same function and advantage as the first through and second embodiments. The artificial heart of the third embodiment, located outside the heart, is less restricted by size than the embedded-type artificial heart, and needs less reliability than the same. However, it is a matter of course that the artificial heart of the third embodiment is also requested to have low sealing liquid consumption and high reliability. The above-described sealing mechanism sufficiently satisfies the requests.

The other structural elements employed in the third embodiment are similar to those in the first and second embodiments. Therefore, they are denoted by corresponding reference numerals in FIG. 7, and their explanation is omitted.

It is also a matter of course that the present invention is not limited to the above-described artificial hearts, but can be applicable to artificial hearts of other types and to an artificial organ having a blood circulating portion, such as an artificial cardiopulmonary organ.

## Claims

1. An artificial organ including a main body, said main body comprising:
(a) a pump section (104);
(b) a driving section (105) having a driving shaft (130, 230) provided on a proximal end of said pump section for driving said pump section via said driving shaft, said driving shaft having an outer peripheral surface;
(c) a sealing mechanism (132) provided between said driving section (105) and said pump section (104), for maintaining said driving shaft in a liquid tight state and preventing blood from entering said driving section (105);
(d) a sealing liquid chamber (137) surrounding said driving shaft (130, 230) between said sealing mechanism (132) and said driving section, said sealing liquid chamber (137) being arranged to be filled with a sealing liquid; and
(e) pump means (135, 136, 140, 250) for feeding said sealing liquid from said sealing liquid chamber to said sealing mechanism
wherein said sealing mechanism (132) contains a mechanical seal (132), said mechanical seal containing a fixed-side seat ring (133) and a rotary-side follow ring (134) with the ends of said seat ring (133) and follow ring (134) in close contact with each other so as to rotate freely, and also contains a pressing mechanism (145, 146, 263, 264) for pressing said follow ring (134) against the seat ring (133) at a specific pressure,
**characterized in that** said pressing mechanism is arranged to press said follow ring (134) against said seat ring (133) by a magnetic force of repulsion or attraction between permanent magnets (145, 146, 263, 264).

2. The artificial organ according to claim 1, **characterized in that** said pump means comprises a dynamic-pressure seal bearing (135, 136, 140) at a distal end of said driving shaft (130, 230), said dynamic-pressure seal bearing functioning as micropump for supplying the sealing liquid from said sealing liquid chamber (137) to said sealing mechanism (132) in small amounts.

3. The artificial organ according to claim 1, **characterized in that** said pump means is a sealing liquid pump (250) provided separately from said pump section (104) and said driving section (105), said sealing liquid pump (250) being connected to said sealing liquid chamber (137) with a flexible tube.

4. The artificial organ according to claim 1, **characterized in that** said mechanical seal (132) contains a hollow bearing tube (131) and said driving shaft (130) supported in said bearing tube so as to rotate freely, said driving shaft being supported by a bearing section (135, 136) within said bearing tube (131) so as to rotate freely concentrically with said bearing tube and be capable of moving in the axis direction, and that said seat ring (133) is formed integrally at the tip end portion of said bearing tube (131), said follow ring (134) is secured integrally to said driving shaft (130), and that said pressing mechanism (145, 146) is arranged to actuate said driving shaft (130) in the axis direction to press said follow ring (134) against said seat ring (133).

5. The artificial organ according to claim 4, **characterized in that** said bearing tube (131) and seat ring (133) integrally formed at the tip end portion of that tube, and said driving shaft (130) and bearing (135, 136) integrally formed on that shaft are formed of ceramic material, and said follow ring (134) is formed of carbon graphite material.

6. The artificial organ according to claim 1, **characterized in that** said mechanical seal (132) contains a hollow bearing tube (131) and said driving shaft (230) supported in said bearing tube so as to rotate freely, said driving shaft being supported by a bearing section (135, 136, 236) within said bearing tube so as to rotate freely concentrically with said bearing tube, and that said seat ring (133) is formed integrally at the tip end portion of said bearing tube (131), said follow ring (134) is provided as a separate member from the driving shaft (230) and positioned at the tip end portion of said driving shaft (230) so as to move freely in the axis direction with respect to the driving shaft, and that said pressing mechanism (263, 264, 270) is arranged to actuate said follow ring (134) in the axis direction with respect to said driving shaft (230) to press said follow ring (134) against said seat ring (133).

7. The artificial organ according to claim 6, **characterized in that** said bearing tube (131) and seat ring (133) integrally formed at the tip end portion of that tube, and said driving shaft (230) and bearing integrally formed on that shaft are formed of ceramic material, and said follow ring (134) is formed of carbon graphite material.

8. The artificial organ according to claim 1, **characterized by** further comprising a sealing liquid bag (112) made of flexible material, filled with said sealing liquid and adapted to be embedded in a human body, said sealing liquid chamber (137) being connected to said sealing liquid bag (112).

9. The artificial organ according to claim 8, **characterized in that** one end portion of a circulating passageway (142) is connected in the vicinity of said mechanical seal (132) in said sealing liquid chamber (137), and the other end portion to said circulating passageway (142) is connected to said sealing liquid bag (112), said sealing liquid being circulated through said sealing liquid chamber (137), circulating passageway (142), and sealing liquid bag (112), and the circulating sealing cooling said mechanical seal (132).

10. The artificial organ according to any one of claims 1 to 8, **characterized by** further comprising a circulating path formed in the main body and having said sealing liquid chamber (137), the sealing liquid being circulated through the circulating path by said pump means.

## Patentansprüche

1. Künstliches Organ mit einem Hauptkörper, wobei der Hauptkörper umfasst:
(a) einen Pumpenabschnitt (104);
(b) einen Antriebsabschnitt (105) mit einer Antriebswelle (130, 230), welcher an einem proximalen Ende des Pumpenabschnitts zum Antreiben des Pumpenabschnitts über die Antriebswelle angeordnet ist, wobei die Antriebswelle eine Außenumfangsoberfläche aufweist;
(c) eine Dichtvorrichtung (132), die zwischen dem Antriebsabschnitt (105) und dem Pumpenabschnitt (104) angeordnet ist, um die Antriebswelle in einem flüssigkeitsdichten Zustand zu halten, und um zu verhindern, dass Blut in den Antriebsabschnitt (105) eintritt;
(d) eine Dichtflüssigkeitskammer (137), welche die Antriebswelle (130, 230) zwischen der Dichtvorrichtung (132) und dem Antriebsabschnitt umgibt, wobei die Dichtflüssigkeitskammer (137) angeordnet ist, um mit einer Dichtflüssigkeit gefüllt zu werden; und
(e) eine Pumpeneinrichtung (135, 136, 140, 250) zum Zuführen der Dichtflüssigkeit von der Dichtflüssigkeitskammer zur Dichtvorrichtung,
wobei die Dichtvorrichtung (132) eine mechanische Dichtung (132) umfasst, wobei die mechanische Dichtung einen Sitzring (133) auf der feststehenden Seite und einen Folgering (134) auf der Drehseite umfasst, wobei die Enden des Sitzrings (133) und des Folgerings (134) in engem Kontakt zueinander sind, um sich frei zu drehen, und sie ebenfalls eine Druckvorrichtung (145, 146, 263, 264) umfasst, um den Folgering (134) bei einem bestimmten Druck gegen den Sitzring (133) zu drücken,
**dadurch gekennzeichnet, dass** die Druckvorrichtung so angeordnet ist, dass sie den Folgering (134) gegen den Sitzring (133) mittels einer magnetischen Abstoßungs- oder Anziehungskraft zwischen Permanentmagneten (145, 146, 263, 264) drückt.

2. Künstliches Organ nach Anspruch 1, **dadurch gekennzeichnet, dass** die Pumpenvorrichtung ein dynamisches Druckdichtlager (135, 136, 140) an einem distalen Ende der Antriebswelle (130, 230) umfasst, wobei das dynamische Druckdichtlager als Mikropumpe zum Zuführen der Dichtflüssigkeit von der Dichtflüssigkeitskammer (137) zur Dichtvorrichtung (132) in kleinen Mengen funktioniert.

3. Künstliches Organ nach Anspruch 1, **dadurch gekennzeichnet, dass** die Pumpenvorrichtung eine Dichtflüssigkeitspumpe (250) ist, die getrennt von dem Pumpenabschnitt (104) und dem Antriebsabschnitt (105) angeordnet ist, wobei die Dichtflüssigkeitspumpe (250) mit der Dichtflüssigkeitskammer (137) über ein flexibles Rohr verbunden ist.

4. Künstliches Organ nach Anspruch 1, **dadurch gekennzeichnet, dass** die mechanische Dichtung (132) ein hohles Lagerrohr (131) und die Antriebswelle (130), die in dem Lagerrohr gelagert ist, um frei zu drehen, umfasst, wobei die Antriebswelle von einem Lagerabschnitt (135, 136) innerhalb des Lagerrohrs (131) gelagert ist, um konzentrisch mit dem Lagerrohr frei zu drehen und.um sich in der Axialrichtung bewegen zu können, und dass der Sitzring (133) einstückig am Spitzendbereich des Lagerrohrs (131) ausgebildet ist, wobei der Folgering (134) einstückig an der Antriebswelle (130) befestigt ist, und dass die Druckvorrichtung (145, 146) so angeordnet ist, dass sie die Antriebswelle (130) in der Axialrichtung in Gang setzt, um den Folgering (134) gegen den Sitzring (133) zu drücken.

5. Künstliches Organ nach Anspruch 4, **dadurch gekennzeichnet, dass** das Lagerrohr (131) und der Sitzring (133), der einstückig am Spitzendbereich des Rohrs ausgebildet ist, und die Antriebswelle (130) und das Lager (135, 136), welches einstückig auf dieser Welle ausgebildet ist, aus keramischem Material gebildet sind, und der Folgering (134) aus Karbongraphitmaterial gebildet ist.

6. Künstliches Organ nach Anspruch 1, **dadurch gekennzeichnet, dass** die mechanische Dichtung (132) ein hohles Lagerrohr (131) und die Antriebswelle (230), die in dem Lagerrohr gelagert ist, um frei zu drehen, umfasst, wobei die Antriebswelle von einem Lagerabschnitt (135, 136, 236) innerhalb des Lagerrohrs gelagert ist, um konzentrisch mit dem Lagerrohr frei zu drehen, und dass der Sitzring (133) einstückig am Spitzendbereich des Lagerrohrs (131) ausgebildet ist, wobei der Folgering (134) als ein getrenntes Element von der Antriebswelle (230) vorgesehen ist und an dem Spitzendbereich der Antriebswelle (230) so positioniert ist, dass er sich frei in der Axialrichtung bezüglich der Antriebswelle bewegen kann, und dass die Druckvorrichtung (263, 264, 270) so angeordnet ist, dass sie den Folgering (134) in der Axialrichtung bezüglich der Antriebswelle (230) in Gang setzt, um den Folgering (134) gegen den Sitzring (133) zu drücken.

7. Künstliches Organ nach Anspruch 6, **dadurch gekennzeichnet, dass** das Lagerrohr (131) und der Sitzring (133), der einstückig am Spitzendbereich des Rohrs ausgebildet ist, und die Antriebswelle (230) und das Lager, welches einstückig auf dieser Welle ausgebildet ist, aus keramischem Material gebildet sind, und der Folgering (134) aus Karbongraphitmaterial gebildet ist.

8. Künstliches Organ nach Anspruch 1, **dadurch gekennzeichnet, dass** es ferner einen Dichtflüssigkeitsbeutel (112) umfasst, hergestellt aus einem flexiblen Material, gefüllt mit der Dichtflüssigkeit und dazu ausgelegt, in einen menschlichen Körper eingebettet zu werden, wobei die Dichtflüssigkeitskammer (137) mit dem Dichtflüssigkeitsbeutel (112) verbunden ist.

9. Künstliches Organ nach Anspruch 8, **dadurch gekennzeichnet, dass** ein Endbereich eines Umlaufdurchgangs (142) in der Nähe der mechanischen Dichtung (132) in der Dichtflüssigkeitskammer (137) verbunden ist, und der andere Endbereich des Umlaufdurchgangs (142) mit dem Dichtflüssigkeitsbeutel (112) verbunden ist, wobei die Dichtflüssigkeit durch die Dichtflüssigkeitskammer (137), den Umlaufdurchgang (142) und den Dichtflüssigkeitsbeutel (112) zirkuliert, und die zirkulierende Dichtflüssigkeit die mechanische Dichtung (132) kühlt.

10. Künstliches Organ nach einem der Ansprüche 1 bis 8, **dadurch gekennzeichnet, dass** es ferner einen Umlaufpfad umfasst, der in dem Hauptkörper ausgebildet ist und eine Dichtflüssigkeitskammer (137) aufweist, wobei die Dichtflüssigkeit durch den Umlaufpfad mittels der Pumpenvorrichtung zirkuliert.

## Revendications

1. Organe artificiel comprenant un corps principal, ledit corps principal se composant de :
(a) une section de pompage (104) ;
(b) une section d'entraînement (105) ayant un arbre d'entraînement (130, 230) situé à une extrémité proximale de ladite section de pompage pour entraîner ladite section de pompage au moyen dudit arbre d'entraînement, ledit arbre d'entraînement ayant une surface périphérique extérieure ;
(c) un mécanisme d'étanchéité (132) situé entre ladite section d'entraînement (105) et ladite section de pompage (104), pour maintenir ledit arbre d'entraînement dans un état étanche aux liquides et empêcher le sang de pénétrer dans ladite section d'entraînement (105) ;
(d) une chambre de liquide d'étanchéité (137). entourant ledit arbre d'entraînement (130, 230) entre ledit mécanisme d'étanchéité (132) et ladite section d'entraînement, ladite chambre de liquide d'étanchéité (137) étant disposée de manière à être remplie par le liquide d'étanchéité ; et
(e) un moyen de pompage (135, 136, 140, 250) pour faire passer ledit liquide d'étanchéité depuis ladite chambre de liquide d'étanchéité vers ledit mécanisme d'étanchéité,
dans lequel ledit mécanisme d'étanchéité (132) contient une garniture mécanique d'étanchéité (132), ladite garniture mécanique d'étanchéité contenant une bague de siège du côté fixe (133) et une bague entraînée du côté rotatif (134) avec les extrémités desdites bague de siège (133) et bague entraînée (134) en contact étroit l'une avec l'autre de manière à tourner librement, et qui contient aussi un mécanisme de pression (145, 146, 263, 264) pour appuyer ladite bague entraînée (134) contre la bague de siège (133) à une pression spécifique,
**caractérisé en ce que** ledit mécanisme de pression est disposé de manière à appuyer ladite bague entraînée (134) contre ladite bague de siège (133) par une force magnétique de répulsion ou d'attraction entre des aimants permanents (145, 146, 263, 264).

2. Organe artificiel selon la revendication 1, **caractérisé en ce que** le moyen de pompage comprend un support d'étanchéité à pression dynamique (135, 136, 140) à une extrémité distale dudit arbre d'entraînement (130, 230), ledit support d'étanchéité à pression dynamique fonctionnant comme une micropompe pour faire passer le liquide d'étanchéité depuis ladite chambre de liquide d'étanchéité (137) vers ledit mécanisme d'étanchéité (132) par petites quantités.

3. Organe artificiel selon la revendication 1, **caractérisé en ce que** ledit moyen de pompage est une pompe à liquide d'étanchéité (250) fournie indépendamment de ladite section de pompage (104) et de ladite section d'entraînement (105), ladite pompe à liquide d'étanchéité (250) étant reliée à ladite chambre de liquide d'étanchéité (137) par un tube souple.

4. Organe artificiel selon la revendication 1, **caractérisé en ce que** ladite garniture mécanique d'étanchéité (132) contient un tube de support creux (131) et ledit arbre d'entraînement (130) maintenu dans ledit tube de support de manière à tourner librement, ledit arbre d'entraînement étant maintenu par une section de support (135, 136) à l'intérieur dudit tube de support (131) de manière à tourner librement de manière concentrique avec ledit tube de support et à pouvoir se déplacer dans la direction de l'axe, et **en ce que** ladite bague de siège (133) est formée en partie intégrante de la portion extrême dudit tube de support (131), ladite bague entraînée (134) est fixée intégralement au dit arbre d'entraînement (130), et **en ce que** ledit mécanisme de pression (145, 146) est disposé de manière à actionner ledit arbre d'entraînement (130) dans la direction de l'axe pour appuyer ladite bague entraînée (134) contre ladite bague de siège (133).

5. Organe artificiel selon la revendication 4, **caractérisé en ce que** lesdits tube de support (131) et bague de siège (133) formée en partie intégrante de la portion extrême de ce tube, et lesdits arbre d'entraînement (130) et support (135, 136) formé en partie intégrante sur cet arbre constitués de matériau céramique, et ladite bague entraînée (134) est constituée de matériau de graphite de carbone.

6. Organe artificiel selon la revendication 1, **caractérisé en ce que** ladite garniture mécanique d'étanchéité (132) contient un tube de support creux (131) et ledit arbre d'entraînement (230) maintenu dans ledit tube de support de manière à tourner librement, ledit arbre d'entraînement étant maintenu par une section de support (135, 136, 236) à l'intérieur dudit tube de support de manière à tourner librement de manière concentrique avec ledit tube de support, et **en ce que** ladite bague de siège (133) formée en partie intégrante de la portion extrême dudit tube de support (131), ladite bague entraînée (134) est fournie comme un élément indépendant de l'arbre d'entraînement (230) et positionnée à la portion extrême dudit arbre d'entraînement de manière à se déplacer librement dans la direction de l'axe par rapport à l'arbre d'entraînement, et **en ce que** ledit mécanisme de pression (263, 264, 270) est disposé pour actionner ladite bague entraînée (134) dans la direction de l'axe par rapport au dit arbre d'entraînement (230) pour appuyer ladite bague entraînée (134) contre ladite bague de siège (133).

7. Organe artificiel selon la revendication 6, **caractérisé en ce que** lesdits tube de support (131) et bague de siège (133) formée en partie intégrante de la portion extrême de ce tube, et lesdits arbre d'entraînement (230) et support formé en partie intégrante sur cet arbre sont constitués de matériau de céramique, et ladite bague entraînée (134) est constituée de matériau de graphite de carbone.

8. Organe artificiel selon la revendication 1, **caractérisé en ce qu'**il comprend en outre une poche de liquide d'étanchéité (112) faite dans un matériau souple, remplie dudit liquide d'étanchéité et adaptée de manière à pouvoir être implantée dans un corps humain, ladite chambre de liquide d'étanchéité (137) étant reliée à ladite poche de liquide d'étanchéité (112).

9. Organe artificiel selon la revendication 8, **caractérisé en ce que** une portion extrême d'un passage de circulation (142) est reliée à proximité de ladite garniture mécanique d'étanchéité (132) dans ladite chambre de liquide d'étanchéité (137), et l'autre portion extrême dudit passage de circulation (142) est reliée à ladite poche de liquide d'étanchéité (112), ledit liquide d'étanchéité étant envoyé à travers lesdits chambre de liquide d'étanchéité (137), passage de circulation (142), et poche de liquide d'étanchéité (112), et ledit liquide d'étanchéité circulant en refroidissant ladite garniture mécanique d'étanchéité (132).

10. Organe artificiel selon l'une quelconque des revendications 1 à 8, **caractérisé en ce qu'**il comprend en outre une voie de circulation formée dans le corps principal et ayant ladite chambre de liquide d'étanchéité (137), le liquide d'étanchéité étant envoyé à travers la voie de circulation par ledit moyen de pompage.
